# EUROPEAN PATENT SPECIFICATION

(11) **EP 1 212 446 B1**
(45) Date of publication and mention of the grant of the patent: **15.04.2009**
(21) Application number: 00963145.8
(22) Date of filing: 08.09.2000
(51) Int. Cl.: C12N 15/86, C12N 15/63, C12N 15/10, C12N 7/04, A61K 39/21, A61K 48/00

(54) **VIRAL REPLICONS AND VIRUSES DEPENDENT ON INDUCING AGENTS**
VIRALE REPLIKONS UND VON INDUZIERENDEN WIRKSTOFFEN ABHÄNGIGE VIREN
REPLICONS VIRAUX ET VIRUS DEPENDANTS D'AGENTS INDUCTEURS

(30) Priority: 10.09.1999 EP 99202971
(43) Date of publication of application: 12.06.2002
(73) Proprietor: Academisch Medisch Centrum bij de Universiteit van Amsterdam, 1105 AZ Amsterdam (NL)
(72) Inventor: BERKHOUT, Benjamin, NL-1412 GH Naarden (NL); VERHOEF, Koenraad, Dirk, Witney, Oxon OX8 5FL (GB)
(74) Representative: Prins, Adrianus Willem
(86) International application number: PCT/NL2000/000637
(87) International publication number: WO 2001/020013

(56) References cited:
- EP-A- 0 494 724
- WO-A-00/37660
- WO-A-01/07637
- WO-A-93/17706
- WO-A-97/00947
- WO-A-97/20463
- WO-A-97/48277
- WO-A-98/17816
- WO-A-98/37185
- WO-A-99/00510
- WO-A-99/31250
- US-A- 5 723 765
- CAPLEN N ET AL: "Adeno-retroviral chimeric viruses as in vivo transducing agents" GENE THERAPY, vol. 6, no. 3, March 1999 (1999-03), pages 454-459, XP000990649
- YAO F ET AL: "A novel tetracycline-inducible viral replication switch" HUM GENE THER, vol. 10, no. 3, 10 February 1999 (1999-02-10), pages 419-427, XP000877364
- HUANG L ET AL: "Human immunodeficiency viruses regulated by alternative trans-activators: genetic evidence for a novel non-transcriptional function of Tat in virion infectivity" EMBO JOURNAL., vol. 13, no. 12, 1916, pages 2886-2896, XP002163801
- VERHOEF K AND BERKHOUT B: "A second-site mutation that restores replication of a Tat-defective human immunodeficiency virus" JOURNAL OF VIROLOGY., vol. 73, no. 4, April 1999 (1999-04), pages 2781-2789, XP002163802
- VERHOEF K ET AL: "Strict control of human immunodeficiency virus type 1 replication by a genetic switch: Tet for Tat" J. VIROL., vol. 75, no. 2, January 2001 (2001-01), pages 979-987, XP002163803

## Description

The present invention relates to the field of molecular biology of pathogens, in particular viruses and more in particular human immuno deficiency virus. It relates to methods for producing replicons and/or viruses dependent on inducing agents, said viruses and/or replicons as well as uses of such replicons and/or viruses in the production of vaccines, in particular live-attenuated vaccines. Live-attenuated virus vaccines (such as vaccinia, polio and measles) have been enormously successful and have made a dramatic and historic impact on public health. However, for the human immunodeficiency virus type 1 (HIV-1) safety concerns remain about either the reversion of attenuated vaccine strains to virulent phenotypes or the induction of fulminant infection in (immunocompromised) individuals. Testifying to the genetic instability of such strains is the recent demonstration that the HIV-1 delta3 vaccine candidate, which contains 3 deletions in non-essential parts of the genome, is able to regain full replication capacity within four months of replication in tissue culture (Berkhout et al., 1999). In addition, it has been reported recently that replication of deletion variants of the simian immunodeficiency virus (SIV) increased after several years in some infected monkeys, concomitant with the onset of AIDS (Baba et al., 1999). Furthermore, although there is some evidence that attenuated HIV-1 variants lacking the *nef* gene result in a benign course of infection in humans (Deacon et al., 1995), a decline in CD4+ T-cell numbers has been reported recently for some of these individuals, which is an early sign that these persons could develop AIDS (Dyer et al., 1999; Greenough et al., 1999). These results have forced the development of subunit or inactivated virus vaccines, but these vaccines have not elicited the potent broad-based immune responses or long-term memory necessary to confer life-long protection in immunized individuals [reviewed in (Paul, 1995)]. It is for this reason that live-attenuated HIV vaccine approaches are still being considered.
Replicating virus vaccines demonstrated superior performance in AIDS vaccine trials. It has been repeatedly demonstrated that macaques or chimpanzees persistently infected with genetically attenuated, non-pathogenic isolates of SIV or HIV-1, respectively, strongly resist a subsequent challenge with pathogenic virus (Shibata et al., 1997; Wyand et al., 1996; van Rompay et al., 1995; Almond et al., 1995; Daniel et al., 1992; Lohman et al., 1994; Stahl-Hennig et al., 1996; Johnson et al., 1999). However, to satisfy safety concerns, the ideal vaccine strain should replicate only to the extent that is needed for immunogenicity. Towards the construction of the next generation of safe, genetically stable HIV-1 variants as live-attenuated AIDS vaccine, we now report the construction of a HIV-1 variant of which the replication depends on the addition of an inducing agent such as the non-toxic, selective effector doxycycline (dox). Thus the invention provides an inducible viral replicon, comprising at least one inducible repressor and/or activator, and all viral sequences which are essential for replication under direct or indirect control of said inducible repressor and/or activator. In one embodiment, at least part of said viral sequences in said inducible replicon is RNA. The invention is exemplified by the preferred embodiments relating to Human Immunodeficiency Virus (HIV). However, the invention will be applicable to other pathogens, in particular viral pathogens, of which it is important that they replicate in order to obtain an efficacious immune response, but for which it is also important that said replication does not go beyond the level required for said immune response. A replicon is defined as a nucleic acid molecule capable of replication in a suitable environment, such as a permissive cell, because it has all the necessary elements for replication in such an environment. We call it a replicon, because it will not always be directly derived from the nucleotide sequences of the original pathogen, for instance in the case of single stranded DNA viruses, RNA viruses, etc. Typically, in order to manipulate nucleic acids, double stranded forms are necessary, typically double stranded DNA forms. Therefore preferred replicons will be double stranded DNA nucleic acids in at least one stage of their life cycle.

A replicon is also intended to reflect that the actual pathogen, or its attenuated live vaccine relative, usually comprises more than just nucleic acid. The nucleic acid is typically packaged into a (viral) particle. Therefore the replicon preferably also encodes a functional packaging signal, allowing for the nucleic acid in its wild-type-like form (RNA in the case of a retrovirus, etc.) to be packed into a viral particle. In order for the replicon to be able to replicate in a host, it is preferred that said replicon also carries the structural genes for the proteins of the envelope and/or capsid, be it in wild-type format or in a somewhat different format (reduced or enhanced target binding, etc.).

In order to be able to regulate the amount of replication necessary for eliciting a good immune response without any replication beyond that level, according to the invention at least one gene essential for said replication is placed under the control of an inducible repressor/activator. In order to prevent leakage, it is preferred to have a combination of essential genes under such control and it is even more preferred to have at least two different repressor/activator combinations in control of at least one, but preferably more than one gene essential for replication. In most (viral) pathogens a number of genes is essential for replication, but most of them also have a sort of "master switch", usually an early gene, usually transactivating other genes. A first candidate to put under direct control of a repressor/activator is of course such a master switch, which then indirectly provides control over the other essential genes for replication. Still it is even then preferred to put at least one other essential gene under control of an inducible repressor/activator. Such a master switch is not required for 'simple' viral genomes such as HIV-1 that are under control of a single transcription unit.

As stated before the replicon is preferably a viral replicon which is derived from a human immunodeficiency virus. Typically such a replicon would be an infectious double stranded DNA clone of an HIV strain. Preferably said HIV strain is already an attenuated strain, or is made into an attenuated strain by introducing mutations, such as functional deletions, e.g. those described herein. Any repressor/activator elements that are inducible are in principle applicable in the present invention. Typically when they are used as a single element they should not have leakage (meaning low base levels of gene expression) in the repressed or unactivated state. In the case of double or more inducible controls, such leakage becomes less important, although essentially no leakage is still highly preferred. A good system for inducible control is the combination of the Tet-operon, together with doxycyclin as the inducing agent. Thus the invention also provides a viral replicon wherein said inducible repressor and/or activator comprises a Tet operon or a functional equivalent thereof. This operon and its necessary elements are as such known and further described herein below. A functional equivalent thereof is an element that is capable of repression and/or activation in essentially the same manner as the Tet operon. Typically this would be highly homologous variations of said operon. As a safety valve, it would be advantageous to provide the replicon with a suicide gene that can be activated when unwanted effects occur such as replication beyond what is necessary for an immune response or rescue by wild type virus, etc. Such a suicide gene is e.g. HSV-tk, which can be induced by adding gancyclovir or a functional equivalent thereof. Upon induction said gene will kill the infected cell, and thereby inhibit further replication and infection of other cells. Thus in yet another preferred embodiment the invention provides a replicon according to the invention which further comprises a suicide gene.

As stated herein before, the replicon is preferably under control of at least a Tet operon, which allows for replication in the presence of doxycyclin. Thus the invention also provides a replicon according to the invention which can be induced to replicate by the presence of doxycyclin or a functional analog thereof.

In the present context a functional analog of doxycyclin is a molecule capable of removing repression or initiating activation of the genes under control of the activator and/or repressor present in the replicon.
In order to attenuate the HIV replicon and/or the resulting virus it is preferred that the replicon is provided with a functional deletion of the TAR-element. Thus in yet another preferred embodiment the invention provides a replicon according to the invention, which further comprises an inactivated TAR element.

In order to attenuate the HIV replicon according to the invention it is preferred to functionally delete the Tat element. Thus the invention also provides a replicon according to the invention, which further comprises an inactivated Tat element. Preferably both elements mentioned above are functionally deleted. Functional deletion means that at least their function in the replication of the replicon is at least partially inhibited. Essential genes for replication typically should not be completely dysfunctional. Proteins necessary for removing repression or initiating activation elements which are present upstream of the essential genes to be put under control should be encoded by the replicon and should be inserted preferably in a non-essential gene. Thus the invention also provides in a preferred embodiment a replicon according the invention wherein at least one functional part, preferably an rtTA gene, of said inducible repressor and/or activator is inserted into the *nef* gene. The functional part in this case of course refers to any proteinaceous substance capable of activating or derepressing the element in control of the essential gene. Preferably space is created for the sequence encoding said proteinaceous substance. Thus the invention also provides a replicon in which at least part of the nef gene is deleted to create space for insertion.
To further attenuate a replicon according to the invention further elements of the wild-type virus can be functionally deleted. Thus the invention further provides a replicon according to the invention, in which at least one NF-kB element has been deleted. It is preferred that the motif to be activated is a tetO motif, preferably present in an LTR. Thus the invention also provides a replicon, which comprises at least one tetO motif in at least one functional LTR. It is preferred to have more than one element before an essential gene. Thus the invention also provides a replicon which comprises at least 2, 4, 6, or 8 such elements in at least one functional LTR.
The LTR is preferably modified to avoid reversion to wild type virus.

The invention further provides methods using the replicons to produce dependent viruses, meaning viruses needing an inducing agent in order to be able to replicate. Thus the invention provides a method for producing a virus dependent on an inducing agent for replication, comprising providing a permissive cell with a replicon according to the invention, culturing said cell in the presence of said inducing agent and harvesting said dependent virus from said culture. Again such methods are preferably applied to HIV. Thus the invention provides a method in which said dependent virus is a human immunodeficiency virus, preferably an attenuated virus.

The preferred inducing agent is again doxycyclin. Thus yet another preferred embodiment is a method in which said inducing agent is doxycyclin or a functional analog thereof. Also part of the present invention are viruses produced by said methods or which can be produced by said methods. Thus the invention also provides a virus dependent on an inducing agent for replication obtainable by a method according to the invention, preferably again a human immunodeficiency virus, again preferably attenuated.

The viruses will find an important application in vaccination. The invention thus also provides a vaccine comprising a replicon according to the invention and/or a virus according to the invention, an amount of inducing agent and optionally a suitable adjuvant.

Of course the vaccine may comprise a single dosage unit, but it may also comprise the inducing agent separately, or it may be made on the spot from a replicon and/or virus that are reconstituted with a liquid excipient such as saline, optionally together with an adjuvant and/or an inducing agent. Viral vaccines are well known in the field. General rules of thumb applicable to known vaccines will also apply to the vaccines of the present invention. Doses will be found through the normal dose finding studies performed during (pre)clinical trials, e.g. by simple titration of the amount of doxycycline as inducing agent. The vaccine may be sufficient on its own, but it may also be used in addition to other vaccines, etc. The inducing agent may be needed over a longer period of time and can then be provided separately. Again the preferred vaccine is one for prophylaxis of infection with a human immunodeficiency virus.
The invention also provides the use of said vaccine in that it provides a method for the prohylaxis of AIDS, comprising administering a vaccine according the invention to a patient and allowing for viral replication for a limited time by providing said inducing agent. Booster vaccinations are possible by simple readdition of the said inducing agent at later times.
The invention also provides a method for the controlled replication of a virus or a viral replicon comprising providing a permissive cell with a replicon or a virus according to the invention, culturing said cell in the presence of said inducing agent and manipulating the amount of inducing agent present.

### Detailed description.

As stated before the replication of a viral replicon was put under control of a repressor and/or activator system. In the examples this was done by incorporation of the Tet-system into the HIV-1 genome.

Several eukaryotic systems for inducible gene expression have been reported, but the Tet-induced regulatory circuit has some unique properties for incorporation into HIV-1 (Gossen et al., 1993; Gossen & Bujard, 1992; Baron et al., 1999). This Tet-system has found wide application and strict and graded regulation of gene expression has been reported in many experimental set-ups, for example, in the breeding of transgenic animals and in gene therapy approaches. Another advantage of these well-characterized regulatory elements from an evolutionary distant organism such as *E. coli* is that one can establish truly monospecific regulatory circuits in higher eukaryotic cells, thereby limiting the danger of unwanted side effects. This system is based on two elements from the *E.coli tet* operon, the tetracycline-inducible repressor protein (TetR) that has been converted into a eukaryotic transcriptional activator (tTA or rtTA), and the tetO operator DNA sequence. We designed a novel strategy to impose regulation on HIV-1 gene expression and replication with the Tet-system, such that an exogenous agent (dox) can be used to reversibly turn on and off viral replication.

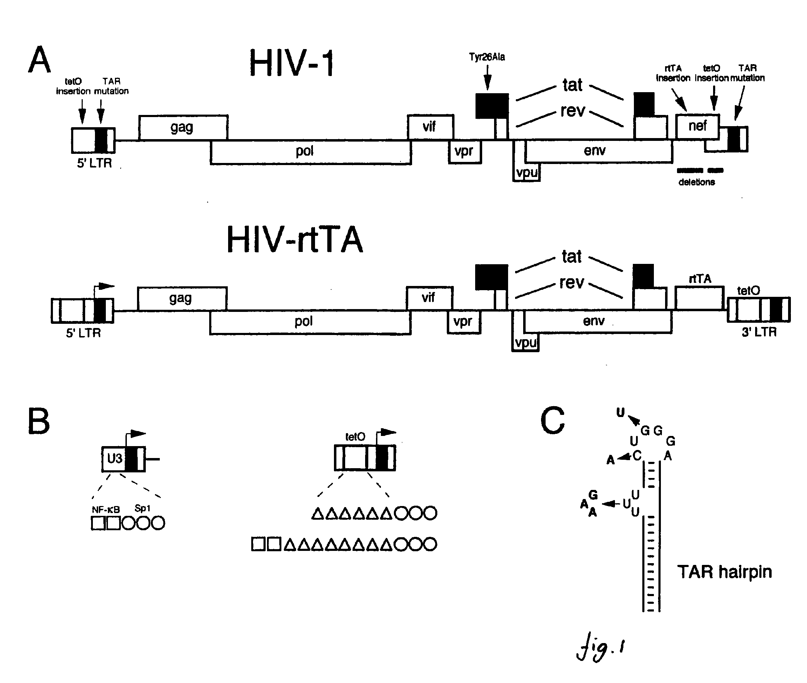

**Construction of HIV-rtTA viruses.** The full-length, infectious HIV-1 molecular clone pLAI was used for construction of an HIV-rtTA virus genome in which the TAR-Tat axis (red in Figure 1A) was replaced by the TetO-rtTA elements (green). In general, we took a conservative approach with regard to the type of mutations that were introduced in the HIV-1 genome in order to minimize the chance to inactivate unknown replicative signals.

TAR and Tat inactivation. First, the TAR element was inactivated by mutation of nucleotides in the single-stranded bulge and loop domains (Figure 1C). Combination of the bulge and loop mutations produces a fully inactive TAR motif because even point mutations in one of these single-stranded TAR domains have a dramatic effect on TAR-function in Tat-mediated LTR transcription and virus replication (Berkhout & Jeang, 1991; Berkhout & Jeang, 1989; Berkhout & Klaver, 1993). We did not introduce more gross sequence changes or even deletions in TAR because this sequence is also essential for virus replication as repeat-R region during strand transfer of reverse transcription (Berkhout et al., 1995). Although we demonstrated previously that the TAR element of the 5'LTR is inherited in both LTRs of the viral progeny, the inactive TAR motif was inserted in both LTRs to minimize the chance of a reversion to the wild-type virus by a recombination event (Klaver & Berkhout, 1994).

Inactivation of the Tat protein was accomplished by introduction of the Tyr26Ala pointmutation. This single amino acid change results in a complete loss of Tat transcriptional activity and viral replication capacity (Verhoef et al., 1997). The corresponding codon change (UAU to GCC) was designed to restrict the likelihood of simple reversion to the wild-type amino acid, which requires at least two substitutions (Verhoef & Berkhout, 1999). It has been suggested that Tat may play additional roles in the replication cycle besides its transcriptional function (Huang et al., 1994; Harrich et al., 1997; Ulich et al., 1999). Thus, Tat may facilitate HIV-rtTA replication even in the absence of an intact TAR element, and we therefore also made viruses with the wild-type tat gene. These constructs will be referred to as **Y** (tyrosine mutant) and **W** (wild-type).

rtTA and tetO insertion. Two deletions were introduced in the *nef* gene create space for the insertion of the non-viral elements (Figure 1A). A 250-nt upstream fragment was removed, and a 200nt fragment overlapping the U3 region of the 3'LTR. This U3-deletion will be inherited by the viral progeny in both LTRs. The exact borders of the U3 and Nef deletions were carefully chosen such that important cis-acting sequences for virus replication were not removed. In particular, we maintained approximately 80-nt around the 5' end of the 3'LTR (Figure 1A). This region encodes multiple sequence elements that are critical for reverse transcription (Ilyinskii & Desrosiers, 1998) and integration (Brown, 1997). In fact, we tried to mimic spontaneous deletions that have been observed in the *nef*/U3 region of several HIV and SIV variants in a variety of replication studies, including in vivo experiments (Kirchhoff et al., 1994; Fisher & Goff, 1998; Ilyinskii et al., 1994; Kirchhoff et al., 1995). As preparation for the insertion of the exogenous rtTA gene into the position of the *nef* gene, a short synthetic sequence was inserted that provides a translational start codon in an optimal sequence context (CCAUGU, (Kozak, 1989) and convenient restriction enzyme recognition sites. The rtTA gene was inserted as *Xcm*I-*Xba*I fragment in this polylinker segment, in frame with the optimized start codon. The splice acceptor that is located just upstream of the *nef* gene was maintained, and rtTA translation should occur from the subgenomic mRNA that was originally meant for expression of the Nef protein.

To identify the optimal configuration of an LTR promoter with rtTA-responsive tetO elements, we first performed transient transfection studies with a variety of LTR-luciferase constructs (Verhoef *et al*., manuscript in preparation). We varied the number of tetO motifs (2, 4, 6, or 8) that were inserted upstream of the three Sp1 binding sites of the HIV-1 LTR promoter. We also tested constructs with and without the two upstream NF-kB elements. The two promoters that provided most robust dox-induced transcription were selected for insertion into the HIV-1 genome and these LTRs are schematically depicted in Figure 1B. They will be referred to as **K** (NF-kB + 8 tetO + Sp1) and **S** (6 tetO + Sp1). Although insertion into the U3 region of the 3'LTR will be sufficient to produce a mutant progeny, we also introduced the tetO motifs in the 5'LTR to generate molecular clones of which the initial round of gene expression in transfected cells is also regulated in a dox-dependent manner. Thus, both LTRs were modified, and this was done in the wild-type and mutant Tat background, resulting in four HIV-rtTA constructs: **KWK, KYK, SWS,** and **SYS.** All HIV-rtTA molecular clones have the TAR inactivation and rtTA insertion in common, but they differ in the status of the tat gene and the type of tetO insert. Of these virus variants, **KWR** is most wild-type-like because it maintained the NF-kB sites and a wild-type Tat protein, and **SYS** is the most minimal HIV-rtTA version.

### HIV-rtTA replicates in a doxycycline-dependent manner.

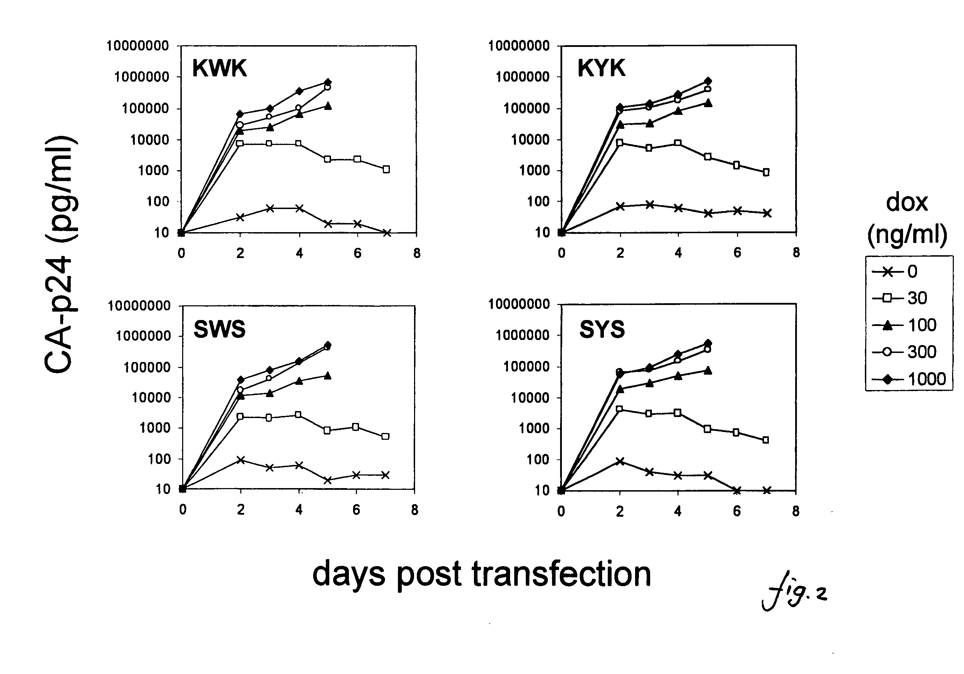

The four pLAI plasmids were individually transfected into the SupT1 T cell line to test for their replication capacity. The culture was maintained at varying dox levels, and virus replication was monitored by measuring the amount of CA-p24 produced in the culture medium (Figure 2). In the presence of optimal dox levels (1000 ng/ml), we measured profound replication of all four HIV-rtTA viruses. No virus replication was observed in the absence of dox, indicating that replication is strictly dependent on the inserted Tet-system. The Tet-system is ideally suited to modulate the level of transcriptional activation in a step-wise manner by reducing the amount of dox (Baron et al., 1997). Indeed, replication of the HIV-rtTA viruses can also be modulated at sub-optimal concentrations of the inducing dox reagent (Figure 2). A progressive reduction in replication rates of all four rtTA-viruses was observed at 300 and 100 ng/ml dox, and virus replication was nearly abolished at 30 ng/ml. These combined results demonstrate that the HIV-rtTA viruses replicate in a strictly dox-dependent manner and that the rate of replication can be fine-tuned by simple variation of the dox-concentration.

The transfected SupT1 cells were killed within 1 week by massive virus-induced syncytia, and CA-p24 production levels reached values that are similar to what is observed in regular infections with the wild-type LAI virus. Nevertheless, the HIV-rtTA variants have a significantly reduced fitness because they showed delayed replication in transfections with less DNA (results not shown). Although the four viruses appear to have a similar replication capacity, this can be measured more appropriately in subsequent infection studies. Indeed, we managed to passage all four HIV-rtTA viruses as cell-free inoculum onto fresh, uninfected T cells, and a spreading infection was sustained for at least 5 weeks (5 passages). From these infection experiments the following ranking order of replication was apparent: **KWK > KYK, SWS > SYS.**

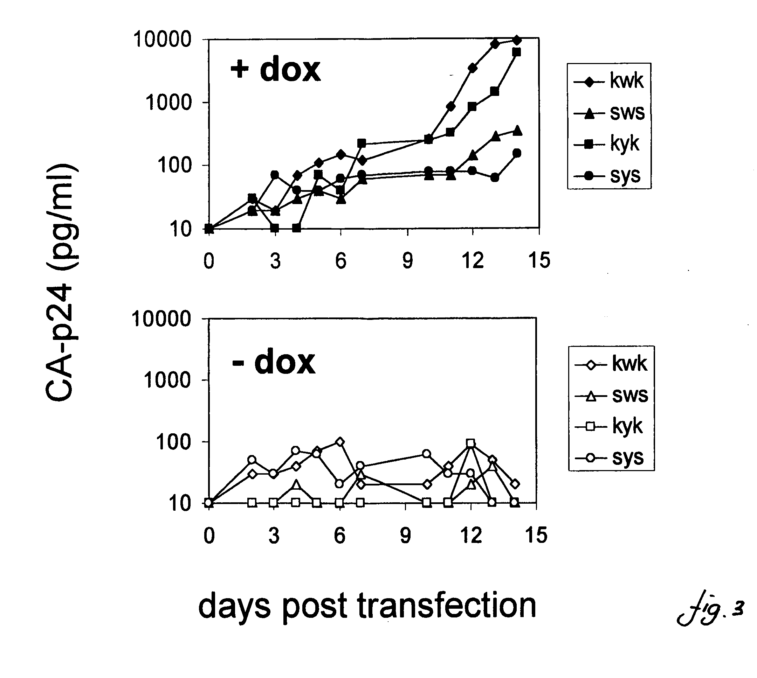

HIV-rtTA vaccine viruses should be able to replicate in primary cells. The LAI molecular clone used in these studies represents a primary isolate that is able to efficiently infect primary cells (Wain-Hobson et al., 1991; Peden et al., 1991), but a complication of our design is that we removed the *nef* gene, which contributes to virus replication in primary cell types (de Ronde et al., 1992). We transfected pooled pheripheral blood mononuclear cells (PBMC) by means of electroporation with 20 µg of the molecular clones and measured CA-p24 production in the culture supernatant for up to two weeks (Figure 3). All four HIV-rtTA variants replicated in the presence of 1000 ng/ml dox, whereas no replication was detectable without dox. The ranking order of replication in PBMCs (**KWK > KYK > SWS > SYS**) is very similar to that observed in SupT1 cells.

### Turning virus replication on and off in a reversible manner.

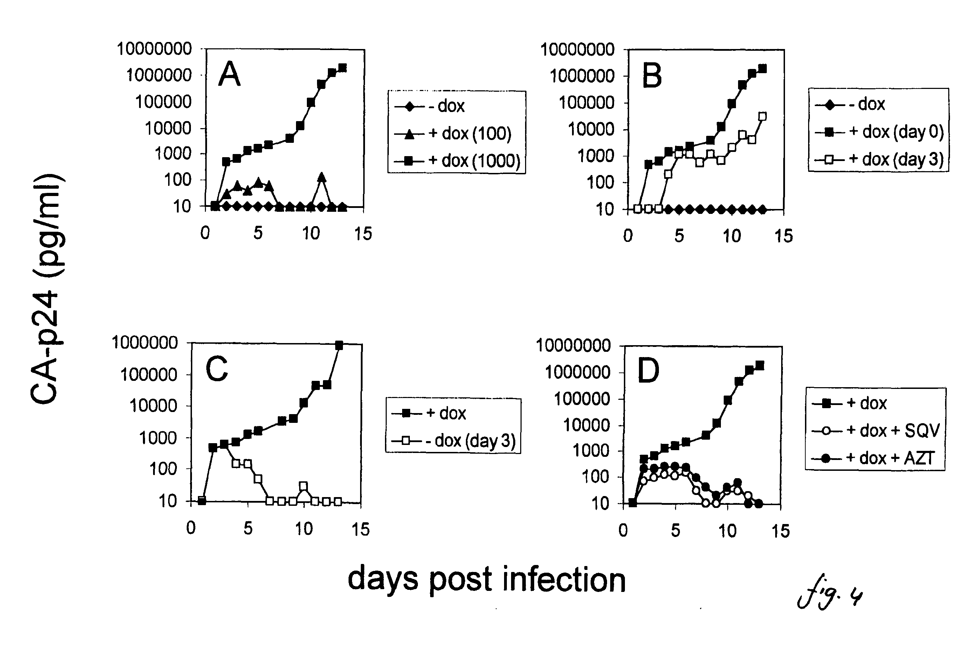

Subsequent tests were performed with the **SWS** virus in SupT1 infections (Figure 4). First, we repeated the dox-response experiment. In this more sensitive infection experiment, it is obvious that the sub-optimal amount of 100 ng/ml dox allows only a low level of replication that is not sufficient to support a spreading infection (Figure 4A). We next analyzed virus replication kinetics when dox was added 3 days after infection of the cells (Figure 4B). This resulted in a delay of virus production of approximately 3 days. In the absence of dox, the HIV-rtTA virus can still infect cells, reverse transcribe its RNA genome and integrate the DNA into the host genome. In other words, the provirus form can be established. This latently infected cell will remain in the culture, and can be activated by dox after three days. An additional feature of the Tet-system is that it provides reversible regulation, and this was tested in the replication assay (Figure 4C). SupT1 cells were infected with the SWS virus and cultured in the presence of dox. At day 3, the cells were washed to remove extracellular dox, and resuspended in medium either with or without dox. Indeed, replication can be stopped abruptly by removal of dox. These combined results confirm that replication of the HIV-rtTA virus is absolutely dependent on dox, and the level of virus replication can be strictly controlled in a graded and reversible manner.

### Safety issues.

Several assays were performed to analyze different safety aspects of the HIV-rtTA variants. First, we screened for leaky virus replication in the absence of dox. For instance, the cell cultures that were transfected with the four different HIV-rtTA constructs (Figure 2) were maintained without dox for a prolonged period of time, but no virus production was measured in these four cultures up to day 52, at which point we stopped the experiment. Similarly, no replicating virus was observed in primary cells without dox (Figure 3). In addition, SupT1 cultures in which virus spread was ongoing in the presence of dox were 'turned off' by removal of dox (see e.g. Figure 4C for the **SWS** virus), without any sign of virus production. These experiments may be viewed as the first safety tests of these vaccine strains.

As an additional safety test, we analyzed the sensitivity of the HIV-rtTA virus to antiretroviral drugs that are in current clinical use. Because we did not alter the basic set of viral genes in HIV-rtTA, including the genes encoding Protease (Pro) and Reverse Transcriptase (RT), these viruses are expected to remain fully sensitive to well-known drugs that target these essential enzymes. As shown in Figure 4D, replication of the dox-dependent **SWS** virus can be inhibited efficiently either by 3'-azido, 3'-deoxythymidine (AZT, a nucleoside RT-inhibitor) or Saquinavir (SQV, a Pro-inhibitor).

### Long-term maintenance of the introduced tetO-rtTA elements.

Several important observations have been made with respect to the safety of the HIV-rtTA designer virus. A key issue is whether the HIV-rtTA virus is genetically stable in terms of maintaining the introduced tetO-rtTA system. We have passaged this virus for a prolonged time (up to 20 weeks in tissue culture), and monitored multiple independent cultures for the status of the inactivated Tat-TAR elements and the introduced rtTA-tetO elements. Sequence analysis revealed no repair of either the Tat protein or the TAR RNA element in any of the cultures. Furthermore, the new rtTA-tetO elements were preserved in all samples. These results, combined with the strict dox-dependency of the cultured viruses, demonstrate that the HIV-rtTA virus retain the introduced transcriptional regulatory system.

Extremely low uninduced HIV-1 expression due to the establishment of an autoregulatory loop. The virus replication experiments indicate that gene expression of HIV-rtTA is strictly dependent on dox, which may come as a surprise because most systems for inducible gene expression, including the original rtTA-system, are known to yield a significant level of 'leaky' expression in the uninduced state. The superior performance of HIV-rtTA may be due, at least in part, to the use of the modified rtTA variant with reduced 'leaky activity'. However, we think that the HIV-rtTA system is different from regular dox-controlled gene expression systems in that an autoregulatory loop has been established that will reduce the level of leaky gene expression. Specifically, we have placed rtTA expression under control of an rtTA-regulated LTR promoter, a situation that mimics the natural autoregulatory loop of the TAR-Tat axis. This means that both the activity of rtTA and its synthesis are dox-dependent. Thus, only minute amounts of rtTA protein will be present in the absence of dox, resulting in an extremely low basal level of gene expression, and consequently a more profound dox-induction. In many other dox-controlled gene expression systems, the tTA or rtTA protein is produced in a constitutive manner from a second locus, e.g. the CMV-rtTA plasmid, which causes a significant level of gene activation in the off-state.

We designed an experiment to critically test whether an autoregulatory loop is established in HIV-rtTA. We mimicked the regular system by co-transfection of HIV-rtTA with CMV-rtTA. The latter plasmid will produce a constitutive level of rtTA protein (even in the absence of dox), which is expected to enhance the level of virus production in the uninduced state. This is indeed what we observed (**Table 1**). The uninduced level of virus production was increased 5- to 10-fold with CMV-rtTA. The results in Table 1 also indicate that additional synthesis of rtTA protein from the co-transfected CMV-rtTA plasmid does not increase the level of virus production in the presence of dox, indicating that all HIV-rtTA constructs are able to produce an optimal amount of rtTA trans-activator. Due to increased basal expression levels in co-transfections with CMV-rtTA, we measured only 8- to 16-fold dox-induction levels. An even more profound dox-effect was measured in the T cell line SupT1 (**Table 2**), ranging from 390- to 3900-fold induction for the different HIV-rtTA constructs. The combined effects of the autoregulatory loop established in HIV-rtTA and the T cell-specific augmentation of the dox-response result in rather dramatic induction levels. In SupT1 cells, an extremely low basal level of virus production is measured, which is estimated to be approximately 0.03% to 0.2 % of the dox-induced state. These results are fully consistent with the inability to measure any virus replication without dox.

### Evolutionary improvement of the Tet-system.

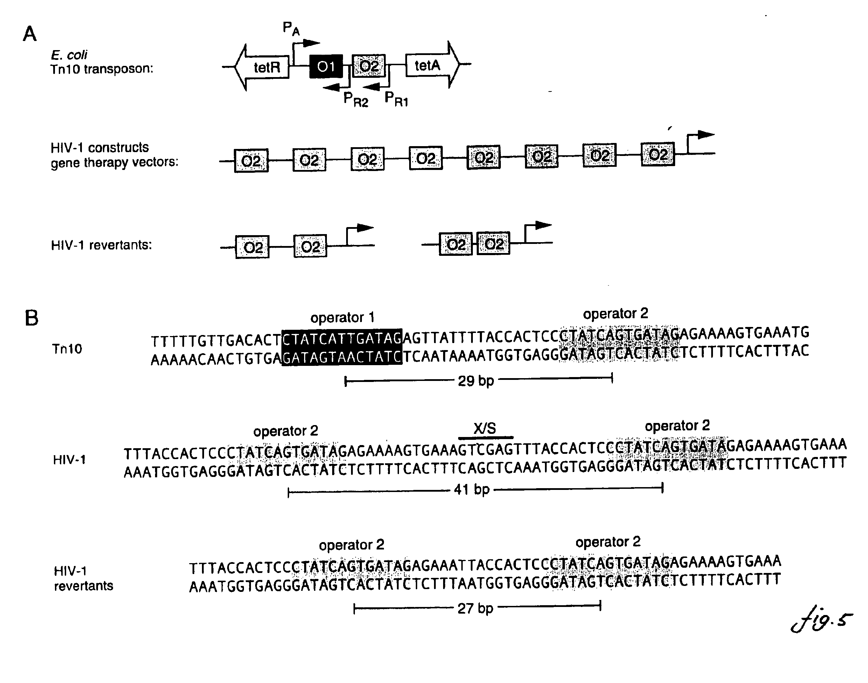

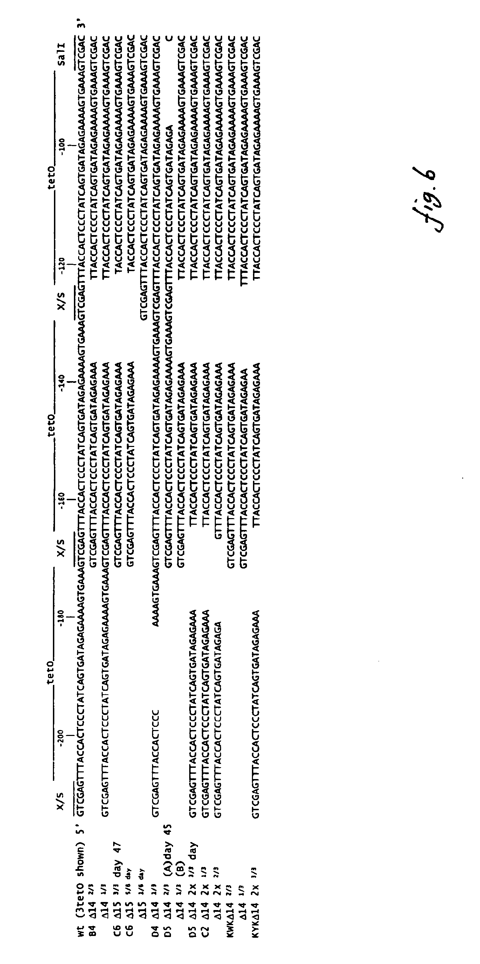

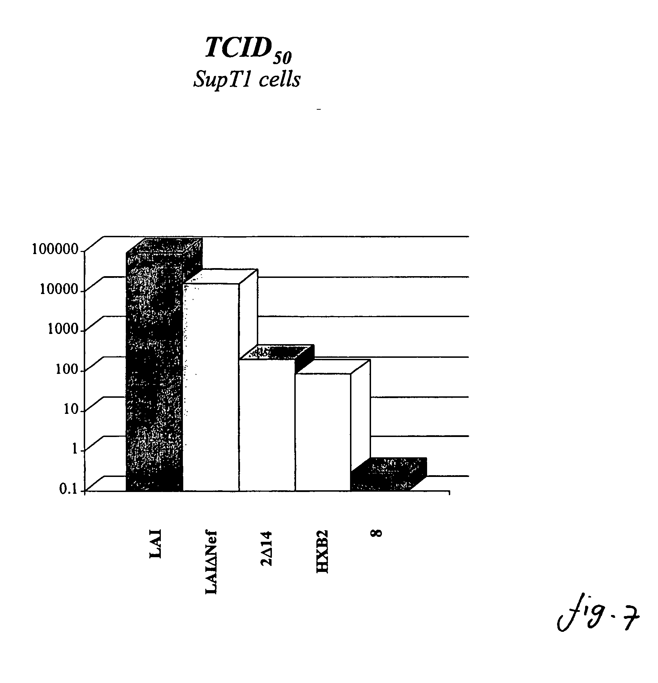

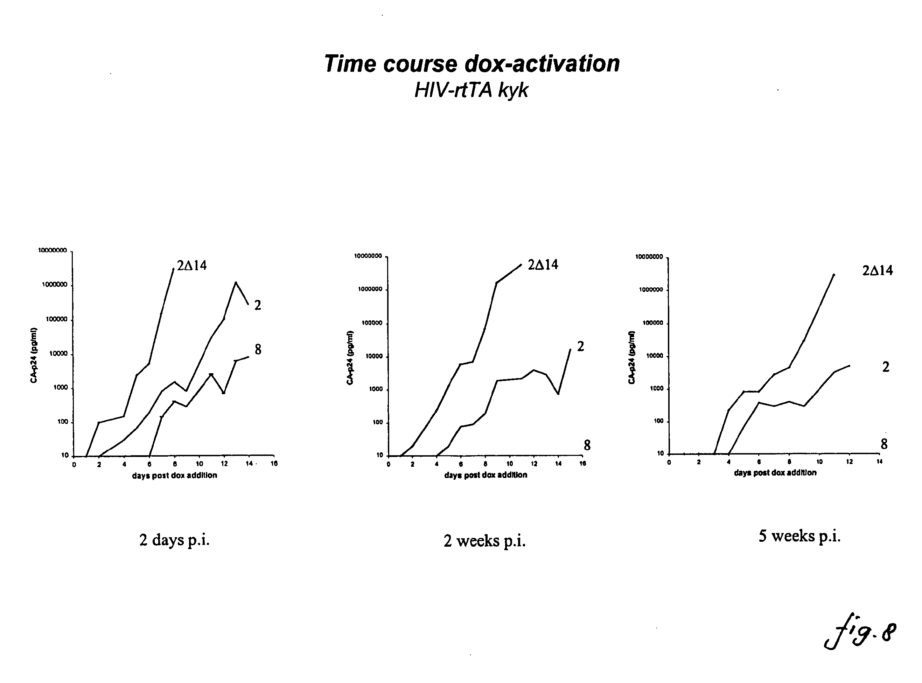

Improved teto configuration. The introduced rtTA-tetO elements can be improved/modified by spontaneous virus evolution. Most strikingly, we frequently observed changes in the number and spacing of the individual tetO motifs in many HIV-rtTA evolution experiments (**Figure 5** **and** **Figure 6**), and we have subsequently shown that these modified promoters are responsible for the significant improvement of virus replication that we witnessed over time (**Figure 7**). The LTR configuration with 2 tetO motifs and altered spacing is most robust as dox-regulated promoter when tested in the context of an integrated provirus. This situation obviously reflects a natural HIV-1 infection, but it also reflects the actual situation of a stably transduced transgene. These findings indicate that we have identified a novel tetO configuration that is optimized for regulated gene expression from a chromosomal position, which is the situation in many gene therapy protocols, transgenic mice etc. Furthermore, whereas the original LTR promoter with 8 tetO elements was rapidly silenced within 2 weeks, we measured sustained activity for the LTR promoter with the optimized tetO elements upon dox-induction (**Figure 8**).

Improved and modified rtTA. Similarly, we have selected for improved versions of the rtTA protein. In long-term cultures of HIV-rtTA, we have observed changes in well-conserved amino acid residues in important protein domains, including the dox-binding site and the DNA binding site. Many properties of this *Escherichia coli* protein are the target for evolutionary improvement, including protein stability in the eukaryotic environment, creation of a nuclear import signal, etc. The improvement that we documented for the tetO motifs demonstrate the enormous potential of this viral evolution approach to improve these signals, and also supports the idea that we are able to select for rtTA variants with a modified effector-specificity. This includes tetracycline-like effector molecules that do not have antibiotic activity.

**The opposite HIV-tTA virus.** We have also constructed the HIV-tTA virus variant, in which the Tat-TAR axis has been replaced by the tTA-tetO system. Again, the replication of this virus is fully dependent on the introduced components of the tetO-rtTA system, but the regulation is opposite to that of HIV-rtTA. The tTA protein is in the DNA-binding conformation without dox, and we measured efficient virus replication in this situation. This virus can be inhibited selectively and specifically by dox, which induces a conformational switch in the tTA protein that abrogates its DNA-binding activity. This HIV-tTA reagent is a useful extension of this approach for certain applications. For instance, the tTA-system is ideally suited for gene therapy approaches that require constitutive expression of the transgene, but providing the option to silence transgene expression at a later time by dox-administration. Obviously, the replicating HIV-tTA reagent will also allow us to improve the tTA reagent by spontaneous virus evolution.

### Discussion.

We have incorporated the Tet-transcriptional system in the HIV-1 genome such that virus replication can now be controlled from the outside by addition of a non-toxic inducer molecule such as doxycycline (dox). Specifically, we constructed replicating HIV-1 variants with inactivating mutations in both arms of the Tat-TAR axis through replacement with the rtTA-tetO elements of the Tet-system. Replication experiments in a T cell line and primary cells convincingly demonstrate that we have successfully designed dox-dependent HIV-1 variants. Replication of these designer HIV-rtTA viruses is regulatable in a graded and reversible manner. Although 'leakiness' has been a problem in some protocols using the rtTA system, we have not observed any virus replication in the absence of dox. One possible explanation is that expression of the rtTA trans-activator in the HIV-rtTA system is fully dependent on the presence of dox. Thus, an autoregulatory loop may have been established that resembles the natural TAR-Tat axis. This mechanism may restrict leakiness or dox-independent replication, thereby providing a significant additional safety feature.

The HIV-rtTA viruses have some unique properties make them ideal reagents for a variety of biological experiments. The most obvious application for such a virus is in the field of live-attenuated vaccines, and a similar approach may be used to put control over other retroviral pathogens (e.g. HIV-2, HTLV-I), pararetroviruses (e.g. HBV), or DNA viruses (e.g. Herpesvirus or Adenovirus). The HIV-rtTA viruses improve the current generation of live-attenuated HIV-1 variants as potential vaccine strains because the conditional replication adds a unique safety feature. The SYS variant has the most minimal 'genotype': TAR⁻, Tat⁻, delta-U3, delta-NF-kB, delta-*nef*, but it should be possible to delete in addition some of the 'accessory' genes such as *vpr*, *vpu* and/or *vif*. HIV-rtTA vaccine viruses should be able to induce a protective immune response, after which replication can be turned off, such that the virus will be stably non-pathogenic. The HIV-rtTA viruses can still be inhibited by antiviral drugs that are in clinical use, and this was demonstrated for the RT-inhibitor AZT and the Pro-inhibitor Saquinavir. These viruses await extensive replication tests to verify their genetic stability, followed by animal tests to screen for their pathogenic potential and their ability to induce a protective immune response.

Because the TAR RNA and tat gene may have become non-essential parts of the HIV-rtTA genome, these elements may now be 'free' to evolve. If these elements have indeed no other function in the viral replication cycle, one would predict that they would eventually be lost by the accumulation of mutations and/or deletion. This further reduces that likelihood of a wild-type-like reversion, thereby making the vaccine strain more safe. However, the situation may be more complex as additional roles have been proposed for both motifs. This is most obvious for the TAR motif, which is part of the R (repeat) region that is critical in strand transfer during reverse transcription. But TAR has also been reported to contribute to RNA packaging in virion particles [reviewed in (Berkhout, 1999)]. The Tat protein has also been implicated in non-transcriptional roles, e.g. during mRNA translation and the process of reverse transcription(SenGupta et al., 1990; Huang, Joshi, Willey, Orenstein, and Jeang, 1994; Harrich, Ulich, Garcia-Martinez, and Gaynor, 1997; Cullen, 1986). Prolonged culture experiments and the analysis of revertant viruses will provide more insight into some of these possibilities.

The HIV-1 TAR-Tat axis was successfully replaced by the tetO-rtTA system, and the latter elements have become essential viral functions. This also adds an important safety feature because it will preclude the spontaneous loss of the new viral elements by deletion, an event that occurs frequently with exogenous sequences that are inserted in a (retro)viral genome. Thus, this feature further enhances the genetic stability of vaccine strains based on HIV-rtTA. On the other hand the current HIV-rtTA variants do not yet replicate optimally, and this is particularly true for the most minimal SYS variant that lacks a functional Tat gene and NF-kB sites. But continued replication has lead to improvement of this new HIV-1 transcriptional axis by selection of spontaneous up-mutants. The beauty of working with HIV, and viruses comprising RNA in general, is that even if a poorly replicating virus is identified, the error-prone nature of the RT enzyme allows for the generation of faster-replicating variants by a method termed forced evolution (Klaver & Berkhout, 1994; Berkhout & Das, 1999). This evolutionary refinement of the initial designer HIV-rtTA variants provides a powerful method to select for fast-replicating, dox-dependent HIV-1 variants. Using this evolutionary approach we have selected for modified forms of the rtTA protein and the tetO sites that are better suited for their new role in virus replication. Thus, the invention also provides a method for modifying an inducible replicon, comprising generating a viral replicon comprising nucleic acid encoding all viral sequences which are essential for replication under direct or indirect control of at least one inducible repressor and/or activator, providing cells, permissive for replication of said replicon, with said replicon, culturing said cells under conditions that allow replication of said replicon, and obtaining replicated replicon from said culture. Said replicon may be derived from an infectious human immunodeficiency virus clone. As described above, this method is well suited for obtaining a modified repressor, activator and/or promoter. Thus the invention also provides a nucleic acid encoding a repressor and/or activator obtainable by said method. The invention also provides a promoter obtainable by said method.

In yet another embodiment, the present invention discloses a cell comprising a replicon of the invention. Said replicon may be modified by a method of the invention described in the preceding paragraph. A cell may also be provided with a modified repressor, activator and/or promoter. Therefore, the invention also discloses a cell comprising a nucleic acid encoding a repressor and/or activator obtainable by said method. The invention also discloses a cell comprising a promoter obtainable by said method.

We expect that we can even use the enormous evolutionary capacity of HIV-1 to select for rtTA elements with altered substrate-specificity by gradually changing dox for other dox-like derivatives in the culture medium. Thus, the virus will help us to find better tetO-rtTA reagents that can subsequently be useful in biological settings that require specific regulation of gene expression (e.g. transgenic mice, gene therapy). We plan to rigorously test the possibility to perform genetics with the 'prokaryotic' Tet-system in this eukaryotic (viral ) background.

Although the novel rtTA-tetO reagents can be used to improve any gene expression system that uses this dox-regulated mechanism, we will specifically discuss the implications for retroviral packaging cell lines and retroviral (gene therapy) vectors. Packaging cell lines based on the HIV-1 lentivirus are notoriously difficult to establish because of toxicity of some viral proteins, and an inducible system is therefore required. We can improve this system at several levels. First, we have made a 1-plasmid construct that expresses both the rtTA protein and the HIV-1 proteins. Second, because of the autoregulatory loop for rtTA synthesis, this system provides an extremely low level of basal activity ('leakiness'), which is an obvious benefit. Third, the LTR promoter with the novel tetO configuration is much more powerful to drive high-level expression. Fourth, this modified LTR is less sensitive to silencing, which is due to chromatin remodelling and /or methylation. The same benefits apply to gene therapy vectors, where improved regulation of transgene expression is critical (either lower basal expression, more robust dox-induced expression, or the absence of transgene-silencing over time). In addition, the tTA-version may be particularly important in long-term transgene expression strategies. Finally, the ability to select for virus variants with rtTA proteins that exhibit either a modified dox-response (e.g. at a lower dox-concentration) or novel effector-specificity may help in the design of additional regulatory circuits that allow the independent regulation of multiple transgenes with different effector molecules.

### REFERENCES

1. Almond N, Kent K, Cranage M, Rud E, Clarke B, Stott EJ. 1995. Protection by attenuated simian immunodeficiency virus in macaques against challenge with virus-infected cells. Lancet 345:1342-4.
2. Baba TW, Liska V, Khimani AH, Ray NB, Dailey PJ, Penninck D, Bronson R, Greene MF, McClure HM, Martin LN, et al. 1999. Live attenuated, multiply deleted simian immunodeficiency virus causes AIDS in infant and adult macaques. Nature Medicine 5:194-203.
3. Baron U, Gossen M, Bujard H. 1997. Tetracycline-controlled transcription in eukaryotes: novel transactivators with graded transactivation potential. Nucleic Acids Res. 25:2723-9.
4. Baron U, Schnappinger D, Helbl V, Gossen M, Hillen W, Bujard H. 1999. Generation of conditional mutants in higher eukaryotes by switching between the expression of two genes. Proc.Natl.Acad.Sci.USA 96:1013-8.
5. Berkhout B. 1999. Multiple biological roles associated with the repeat (R) region of the HIV-1 RNA genome. Adv. Pharmacol. in press*:*
6. Berkhout B, Das AT. 1999. Functional analysis of RNA signals in the HIV-1 genome by forced evolution. In: 7. Barciszewski J, Clark BFC, eds. RNA biochemistry and biotechnology. Dordrecht, the Netherlands.: Kluwer Academic Publishers; pp. in press
8. Berkhout B, Jeang KT. 1989. Trans activation of human immunodeficiency virus type 1 is sequence specific for both the single-stranded bulge and loop of the trans-acting-responsive hairpin: a quantitative analysis. J.Virol. 63:5501-4.
9. Berkhout B, Jeang KT. 1991. Detailed mutational analysis of TAR RNA: critical spacing between the bulge and loop recognition domains. Nucleic Acids Res. 19:6169-76.
10. Berkhout B, Klaver B. 1993. In vivo selection of randomly mutated retroviral genomes. Nucleic Acids Res. 21:5020-4.
11. Berkhout B, van Wamel J, Klaver B. 1995. Requirements for DNA strand transfer during reverse transcription in mutant HIV-1 virions. J.Mol.Biol. 252:59-69.
12. Berkhout B, Verhoef K, van Wamel JLB, Back B. 1999. Genetic instability of live-attenuated HIV-1 vaccine strains. J.Virol. 73:1138-45.
13. Brown PO. 1997. Integration. In: Coffin JM, Hughes SH, Varmus HE, eds. Retroviruses. New York: Cold Spring Harbor Laboratory Press; pp. 161-204.
14. Cullen BR. 1986. Trans-activation of human immunodeficiency virus occurs via a bimodal mechanism. Cell 46:973-82.
15. Daniel MD, Kirchhoff F, Czajak SC, Sehgal PK, Desrosiers RC. 1992. Protective effects of a live attenuated SIV vaccine with a deletion in the nef gene. Science 258:1938-41. de Ronde A, Klaver B, Keulen W, Smit L, Goudsmit J. 1992. Natural HIV-1 NEF accelerates virus replication in primary human lymphocytes. Virol. 178:1-5.
16. Deacon NJ, Tsykin A, Solomon A, Smith K, Ludford-Menting M, Hooker DJ, McPhee DA, Greenway AL, Ellett A, Chatfield C, et al. 1995. Genomic structure of an attenuated quasi species of HIV-1 from blood transfusion donor and recipients. Science 270:988-91.
17. Dingwall C, Ernberg I, Gait MJ, Green SM, Heaphy S, Karn J, Lowe AD, Singh M, Skinner MA, Valerio R. 1989. Human Immunodeficiency Virus 1 tat protein binds trans-activating-responsive region (TAR) RNA in vitro. Proc.Natl.Acad.Sci.USA 86:6925-9.
18. Dyer WB, Ogg GS, Demoitie M-A, Jin X, Geczy AF, Rowland-Jones SL, McMichael AJ, Nixon DF, Sullivan JS. 1999. Strong human immunodeficiency virus (HIV)-specific cytotoxic T-lymphocyte activity in Sydney blood bank cohort patients infected with nef-defective HIV type 1. J.Virol. 73:436-43. Fisher J, Goff SP. 1998. Mutational analysis of stem-loops in the RNA packaging signal of the Moloney murine leukemia virus. Virol. 244:133-45.
19. Gossen M, Bonin AL, Bujard H. 1993. Control of gene activity in higher eukaryotic cells by prokaryotic regulatory elements. Trends Biochem.Sci. 18:471-5.
20. Gossen M, Bujard H. 1992. Tight control of gene expression in mammalian cells by tetracycline-responsive promoters. Proc.Natl.Acad.Sci.USA 89:5547-51.
21. Greenough TC, Sullivan JL, Desrosiers RC. 1999. Declining CD4 T-cell counts in a person infected with nef-deleted HIV-1. New Engl.J.Med. 340:236-7.
22. Harrich D, Ulich C, Garcia-Martinez LF, Gaynor RB. 1997. Tat is required for efficient HIV-1 reverse transcription. EMBO J. 16:1224-35.
23. Huang LM, Joshi A, Willey R, Orenstein J, Jeang KT. 1994. Human immunodeficiency viruses regulated by alternative trans- activators: genetic evidence for a novel non-transcriptional function of Tat in virion infectivity. EMBO J. 13:2886-96.
24. Ilyinskii PO, Daniel MD, Simon MA, Lackner AA, Desrosiers RC. 1994. The role of the upstream U3 sequences in the pathogenesis of simian immunodeficiency virus-induced AIDS in rhesus monkeys. J.Virol. 68:5933-44.
25. Ilyinskii PO, Desrosiers RC. 1998. Identification of a sequence element immediately upstream of the polypurine tract that is essential for replication of simian immunodeficiency virus. EMBO J. 17:3766-74.
26. Johnson RP, Lifson JD, Czajak SC, Stefano cole K, Manson KH, Glickman RL, Yang JQ, Montefiori DC, Montelaro RC, Wyand MS, et al. 1999. Highly attenuated vaccine strains of simian immunodeficiency virus protect against vaginal challenge: inverse relationship of degree of protection with level of attenuation. J.Virol. 73:4952-61.
27. Kirchhoff F, Greenough T, Brettler DB, Sullivan JL, Desrosiers RC. 1995. Absence of intact nef sequences in a long-term survivor with nonprogressive HIV-1 infection. New Engl.J.Med. 332:228-32.
28. Kirchhoff F, Kestler III HW, Desrosiers RC. 1994. Upstream U3 sequences in simian immunodeficiency virus are selectively deleted in vivo in the absence of an intact nef gene. J.Virol. 68:2031-17.
29. Klaver B, Berkhout B. 1994. Evolution of a disrupted TAR RNA hairpin structure in the HIV-1 virus. EMBO J. 13:2650-9. Klaver B, Berkhout B. 1994. Premature strand transfer by the HIV-1 reverse transcriptase during strong-stop DNA synthesis. Nucleic Acids Res. 22:137-44.
30. Kozak M. 1989. The scanning model for translation: an update. J.Cell Biol. 108:229-41.
31. Lohman BL, McChesney MB, Miller CJ, McGowan E, Joye SM, van Rompay KK, Reay E, Antipa L, Pedersen NC, Marthas ML. 1994. A partially attenuated simian immunodeficiency virus induces host immunity that correlates with resistance to pathogenic virus challenge. J.Virol. 68:7021-9.
32. Paul WE. 1995. Can the immune response control HIV infection? Cell 82:177-82.
33. Peden K, Emerman M, Montagnier L. 1991. Changes in growth properties on passage in tissue culture of viruses derived from infectious molecular clones of HIV-1LAI, HIV-1MAL, and HIV-1ELI. Virol. 185:661-72.
34. SenGupta DN, Berkhout B, Gatignol A, Zhou AM, Silverman RH. 1990. Direct evidence for translational regulation by leader RNA and Tat protein of human immunodeficiency virus type 1. Proc.Natl.Acad.Sci.USA 87:7492-6.
35. Shibata R, Siemon C, Czajak SC, Desrosiers RC, Martin MA. 1997. Live, attenuated simian immunodeficiency virus vaccines elicit potent resistance against a challenge with a human immunodeficiency virus type 1 chimeric virus. J.Virol. 71:8141-8.
36. Stahl-Hennig C, Dittmer U, Nisslein T, Petry H, Jurkiewicz E, Fuchs D, Wachter H, Matz-Rensing K, Kuhn EM,
37. Kaup FJ, et al. 1996. Rapid development of vaccine protection in macaques by live-attenuated simian immunodeficiency virus. J.Gen.Virol. 77:2969-81.
38. Ulich C, Dunne A, Parry E, Hooker CW, Gaynor RB, Harrich D. 1999. Functional domains of Tat required for efficient human immunodeficiency type 1 reverse transcription. J.Virol. 73:2499-508.
39. van Rompay KKA, Spinner A, Otsyula M, McChesney MB, Marthas ML. 1995. Attenuated retrovirus vaccines and AIDS. Science 270:1218
40. Verhoef K, Berkhout B. 1999. A second-site mutation that restores replication of a Tat-defective human immunodeficiency virus. J.Virol. 73:2781-9.
41. Verhoef K, Koper M, Berkhout B. 1997. Determination of the minimal amount of Tat activity required for human immunodeficiency virus type 1 replication. Virol. 237:228-36.
42. Wain-Hobson S, Vartanian J-P, Henry M, Chenciner N, Cheynier R, Delassus S, Pedroza Martins L, Sala M, Nugeyre MT, Guétard D, et al. 1991. LAV revisited: origins of the early HIV-1 isolates from Institut Pasteur. Science 252:961-5.
43. Wei P, Garber ME, Fang S-M, Fisher WH, Jones KA. 1998. A novel CDK9-associated C-type cyclin interacts directly with HIV-1 Tat and mediates its high-affinity, loop-specific binding to TAR RNA. Cell 92:451-62.
44. Wyand MS, Manson KH, Garcia-Moll M, Montefiori D, Desrosiers RC. 1996. Vaccine protection by a triple deletion mutant of simian immuodeficiency virus. J.Virol. 70:3724-33.

### Legends to the figures

**Figure 1****. Design of a Tetracycline-dependent HIV-1. Panel A** shows the HIV-1 genome and multiple modifications that were introduced to construct HIV-rtTA. Details of the mutations are provided in the text, see also panels B and C for the LTR modifications. In brief, we inactivated the TAR-Tat transcriptional axis (marked in red) and replaced it by the Tetracycline-inducible tetO-rtTA system (marked in green). Inactivation of the TAR and Tat is marked by crosses through the motifs. The genome maps are not drawn to scale, but the genome size of HIV-rtTA is larger than that of HIV-1. The RNA genome of HIV-1 LAI is 9229-nt, and HIV-rtTA is either 9767-nt (the **S.S** variants) or 9875-nt (**K.K** variants). **Panel B** provides some details of the tetO insertions in the LTR promoter. The U3 region of the wild-type LTR (left) encodes 2 NF-kB sites (squares) and 3 Sp1 sites (circles). The modified LTR (right) contains either 6 or 8 tetO operators (green triangles) upstream of the Sp1 sites. The 6 tetO variant only has the Sp1 sites in mutant **S,** whereas both NF-kB sites are present upstream of the 8 tetO operators in mutant **K.** The arrow marks the transcription start site at the U3-R border, which also is the start site of the TAR hairpin. **Panel C** shows the TAR hairpin structure and the inactivating mutations that were introduced in the bulge (triple-nucleotide substitution) and in the loop (two point mutations). These mutations should disrupt binding of the viral Tat protein and the cellular cyclin T co-factor, respectively (Dingwall et al., 1989; Wei et al., 1998).
**Figure 2****. Doxycycline-controlled replication of the HIV-rtTA viruses.** The SupT1 T cell line was electroporated with 10 µg of the indicated molecular clones, and cells were cultured in without or with an increasing concentration of dox (0 to 1000 ng/ml range). Virus production was measured by CA-p24 elisa on culture supernatant samples.
**Figure 3****. Doxycycline-dependent replication of HIV-rtTA viruses in primary cells.** PBMCs were electroporated with the four individual HIV-rtTA constructs (20 µg), and the cultures were maintained without or with (1000 ng/ml) dox. Fresh uninfected cells were added immediately after transfection and at day 6 post infection. Virus production was measured by CA-p24 elisa on culture supernatant samples.
**Figure 4****. Replication of HIV-rtTA can be turned on and turned off.** These experiments were performed with the **SWS** virus, but similar results have been obtained with the other HIV-rtTA variants. We used the SWS virus (2200 ng CA-p24) to infect 6 x 10⁶ SupT1 cells at day 0. **Panel A** shows the replication potential with 0, 100 and 1000 ng/ml dox. In **panel B,** we analyzed the effect of delayed addition of dox (1000 ng/ml) at day 3 after infection. In **panel C,** the infected cells were grown in 1000 ng/ml dox for 3 days, at which point the cells were washed and incubated in the absence or presence of dox. In **panel D,** infected cells were maintained in the presence dox and we tested the effect of the Protease-inhibitor Saquinavir (200 nM) and the RT-inhibitor AZT (1 µg).
**Figure 5****. Overview of the original and evolved tetO configuration.** We show the natural situation in *Escherichia coli*, the situation in most dox-controlled gene expression cassettes with multiple tetO motifs (8x in HIV-rtTA), and the configurations that were selected by spontaneous virus evolution. The latter form either has 2 tetO motifs or 2 tetO motifs with an altered spacing (see Figure 6 for further details).
**Figure 6****. Sequence of the modified tetO configuration that was selected by spontaneous virus evolution.** The wild-type (wt) sequence of 3 tetO motifs is shown on top. Most viruses evolve from having 8 to 2 tetO motifs (Figure 5). The virus cultures are listed that showed a deletion in the tetO-region. A 14-bp deletion was seen in 6 cultures, and a 15-bp deletion was observed in the C6 culture. Most strikingly, all deletions remove the tetO-spacer element.
**Figure 7****. The titer of HIV-1 variants with a different tetO configuration.** We determined the tissue culture infectious dose (TCID50) on the SupT1 T cell line for several viruses: wild-type HIV-1 (LAI isolate), a nef-deleted LAI variant (LAIΔnef), HIV-rtTA with 2 tetO motifs and altered spacing (2Δ14), the HXB2 isolate (vpr/vpu/nef-minus), and the original HIV-rtTA construct with 8 tetO motifs. The change from the 8 to 2Δ14 tetO configuration improves the virus titer approximately 100-fold. On the other hand, wt LAI is 100/1000-fold better than 2Δ14. It is therefore likely that further improvement of 2Δ14 will take place. In fact, we have selected HIV-rtTA variants that replicate comparable to wild-type LAI, and the observed rtTA changes (see the text) are likely to be reponsible, at least in part, for this.
**Figure 8****. The improved tetO configuration allows long-term gene expression.** The T cell line SupT1 was infected with HIV-rtTA with a different tetO configuration (8, 2 and 2Δ14). Gene expression was induced with dox at different times postinfection (day 2, weeks 2 and 5). The 8 tetO virus demonstrates complete silencing within 2 weeks. The 2tetO virus, and in particular the 2Δ14 virus, exhibit sustained activity. We have been able to induce the 2Δ14 virus up to 12 weeks postinfection (not shown).

**Table 1. Transient HIV-rtTA production in C33A cells (ρg/ml CA-p24)**

| | | | | | + CMV-rtTA | |
|---|---|---|---|---|---|---|
| | | | fold | | | fold |
| | - dox | + dox | induction | - dox | + dox | induction |
| **KWK** | 11,600 | 545,000 | 47 x | 91,500 | 715,000 | 7.8 x |
| **SWS** | 11,350 | 560,000 | 49 x | 49,500 | 560,000 | 11.3 x |
| **KYK** | 4,850 | 580,000 | 120 x | 47,500 | 500,000 | 10.5 x |
| **SYS** | 5,950 | 455,000 | 76 x | 32,500 | 520,000 | 16 x |
| **LAI** | 635,000 | 520,000 | 0.8 x | - | - | - |

**Table 2. Transient HIV-rtTA production in SupT1 cells (pg/ml CA-p24)**

| | | | fold |
|---|---|---|---|
| | - dox | + dox | induction |
| **KWK** | 110 | 54,000 | 491 x |
| **SWS** | 30 | 65,000 | 2167 x |
| **KYK** | 10 | 39,000 | 3900 x |
| **SYS** | 20 | 7,800 | 390 x |

## Claims

1. An inducible human immunodeficiency virus replicon of which the replication is regulated by an exogenous agent, comprising a nucleic acid sequence comprising at least one inducible repressor and/or activator, and all viral sequences which are essential for replication under direct or indirect control of said inducible repressor and/or activator.

2. An inducible replicon according to claim 1, wherein at least part of said viral sequences is RNA.

3. A viral replicon according to claim 2 which is derived from an infectious human immunodeficiency virus clone.

4. A viral replicon according to any one of claims 1-3, wherein said inducible repressor and/or activator comprises a Tet operon or a functional equivalent thereof.

5. A replicon according to any one of claims 1-4 which encodes an attenuated virus.

6. A replicon according to any one of claims 1-5 which further comprises a suicide gene.

7. A replicon according to any one of claims 1-6, which can be induced to replicate by the presence of doxycyclin or a functional analog thereof.

8. A replicon according to any one of claims 3-7, which further comprises an inactivated TAR element.

9. A replicon according to any one of claims 3-8, which further comprises an inactivated Tat element.

10. A replicon according to any one of claims 3-7, wherein at least one functional part, preferably an rtTA gene, of said inducible repressor and/or activator is inserted into the nef gene.

11. A replicon according to claim 10 in which at least part of the nef gene is deleted to create space for insertion.

12. A replicon according to any one of claims 3-11, in which at least one NF-kB element has been deleted.

13. A replicon according to any one of claims 3-12, which comprises at least one tetO motif in at least one functional LTR.

14. A replicon according to claim 13, which comprises at least 2, 4, 6, or 8 such elements in at least one functional LTR.

15. A replicon according to any one of claims 3-14, wherein at least one LTR is modified to avoid reversion to wild type virus.

16. A method for producing a human immunodeficiency virus dependent on an inducing agent for replication, comprising providing a permissive cell with a replicon according to anyone of claims 1-15, culturing said cell in the presence of said inducing agent and harvesting said dependent virus from said culture.

17. A method according to claim 16, in which said virus is an attenuated virus.

18. A method according to anyone of claims 16-17, in which said inducing agent is doxycyclin or a functional analog thereof.

19. A virus dependent on an inducing agent for replication obtainable by a method according to any one of claims 16-18.

20. A virus according to claim 19 which is an attenuated virus.

21. A vaccine comprising a replicon according to any one of claims 1-15 and/or a virus according to any one of claims 19-20, an amount of inducing agent and a suitable adjuvant.

22. A vaccine according to claim 21 whereby said inducing agent is provided separately.

23. A method for the controlled replication of a virus or a viral replicon comprising providing a permissive cell with a replicon according to any one of claims 1-15, or a virus according to claim 19 or 20, culturing said cell in the presence of said inducing agent and manipulating the amount of inducing agent present.

24. A replicon according to any one of claims 1-15 and/or a virus according to any one of claims 19-20 for use as a prophylactic agent.

25. Use of a replicon according to any one of claims 1-15 and/or a virus according to any one of claims 19-20 for the preparation of a vaccine for the prophylaxis of AIDS.

26. A method for modifying an inducible replicon according to anyone of claims 1-15, comprising
- generating a human deficiency virus replicon comprising nucleic acid encoding all viral sequences which are essential for replication under direct or indirect control of at least one inducible repressor and/or activator,
- providing cells, permissive for replication of said replicon, with said replicon,
- culturing said cells under conditions that allow replication of said replicon,
- obtaining replicated replicon from said culture.

27. A cell comprising a replicon according to anyone of claims 1-15.

28. A cell comprising a virus according to any one of claims 19-20.

## Patentansprüche

1. Induzierbares Replikon eines humanen Immundefizienz-Virus, bei dem die Replikation durch ein exogenes Mittel reguliert wird, und das eine Nukleinsäuresequenz umfasst, welche mindestens einen induzierbaren Repressor und/oder Aktivator umfasst, sowie alle viralen Sequenzen, die für die Replikation unter direkter oder indirekter Kontrolle des induzierbaren Repressors und/oder Aktivators erforderlich sind.

2. Induzierbares Replikon nach Anspruch 1, bei dem mindestens ein Teil der viralen Sequenzen RNA ist.

3. Virale Replikon nach Anspruch 2, das von einem infektiösen Klon eines humanen Immundefizienz-Virus abgeleitet ist.

4. Virales Replikon nach einem der Ansprüche 1 bis 3, bei dem der induzierbare Repressor und/oder Aktivator ein Tet-Operon oder ein funktionelles Äquivalent davon umfasst.

5. Replikon nach einem der Ansprüche 1 bis 4, das für ein attenuiertes Virus kodiert.

6. Replikon nach einem der Ansprüche 1 bis 5, das ferner ein Suicide-Gen umfasst.

7. Replikon nach einem der Ansprüche 1 bis 6, das in Gegenwart von Doxycyclin oder einem funktionellen Analog davon induziert werden kann, so dass es repliziert wird.

8. Replikon nach einem der Ansprüche 3 bis 7, das ferner ein inaktiviertes TAR-Element umfasst.

9. Replikon nach einem der Ansprüche 3 bis 8, das ferner ein inaktiviertes Tat-Element umfasst.

10. Replikon nach einem der Ansprüche 3 bis 7, bei dem mindestens ein funktioneller Teil, vorzugsweise ein rtTA-Gen, des induzierbaren Repressors und/oder Aktivators in das nef-Gen insertiert ist.

11. Replikon nach Anspruch 10, bei dem mindestens ein Teil des nef-Gens deletiert ist, so dass Platz für die Insertion erzeugt wurde.

12. Replikon nach einem der Ansprüche 3 bis 11, bei dem mindestens ein NF-kB-Element deletiert wurde.

13. Replikon nach einem der Ansprüche 3 bis 12, das mindestens ein tetO-Motiv in mindestens einem funktionellen LTR umfasst.

14. Replikon nach Anspruch 13, das mindestens 2, 4, 6 oder 8 solcher Elemente in mindestens einem funktionellen LTR umfasst.

15. Replikon nach einem der Ansprüche 3 bis 14, bei dem mindestens ein LTR modifiziert ist, um eine Reversion zum Wildtyp-Virus zu verhindern.

16. Verfahren zur Herstellung eines humanen Immundefizienz-Virus, das hinsichtlich der Replikation abhängig von einem induzierenden Mittel ist, bei dem man eine dazu geeignete Zelle mit einem Replikon nach einem der Ansprüche 1 bis 15 ausstattet, die Zelle in Gegenwart des induzierenden Mittels kultiviert, und das abhängige Virus aus der Kultur erntet.

17. Verfahren nach Anspruch 16, bei dem das Virus ein attenuiertes Virus ist.

18. Verfahren nach einem der Ansprüche 16 bis 17, bei dem das induzierende Mittel Doxycyclin oder ein funktionelles Analog davon ist.

19. Virus, das hinsichtlich seiner Replikation von einem induzierenden Mittel abhängig ist, und das durch ein Verfahren nach einem der Ansprüche 16 bis 18 erhältlich ist.

20. Virus nach Anspruch 19, bei dem es sich um attenuiertes Virus handelt.

21. Impfstoff, der eine Replikon nach einem der Ansprüche 1 bis 15 und/oder ein Virus nach einem der Ansprüche 19 bis 20, eine Menge des induzierenden Mittels und ein geeignetes Adjuvans umfasst.

22. Impfstoff nach Anspruch 21, bei dem das induzierende Mittel getrennt bereitgestellt wird.

23. Verfahren für die kontrollierte Replikation eines Virus oder eines viralen Replikons, bei dem man eine dazu geeignete Zelle mit einem Replikon nach einem der Ansprüche 1 bis 15 ausstattet, oder mit einem Virus nach den Ansprüchen 19 oder 20, die Zelle in Gegenwart des induzierenden Mittels kultiviert, und die Menge des vorhandenen induzierenden Mittels manipuliert.

24. Replikon nach einem der Ansprüche 1 bis 15 und/oder Virus nach einem der Ansprüche 19 bis 20, zur Verwendung als prophylaktisches Mittel.

25. Verwendung eines Replikons nach einem der Ansprüche 1 bis 15 und/oder eines Virus nach einem der Ansprüche 19 bis 20 für die Herstellung eines Impfstoffs für die Prophylaxe gegen AIDS.

26. Verfahren zur Modifizierung eines induzierbaren Replikons nach einem der Ansprüche 1 bis 15, bei dem man
- ein Replikon eines humanen Immundefizienz-Virus erzeugt, das Nukleinsäure umfasst, welche für die gesamte virale Sequenz kodiert, die für die Replikation unter der direkten oder indirekten Kontrolle mindestens eines induzierbaren Repressors und/oder Aktivators erforderlich ist,
- Zellen, die für die Replikation des Replikons geeignet sind, mit dem Replikon ausstattet,
- die Zellen unter Bedingungen kultiviert, welche die Replikation des Replikons erlauben,
- das replizierte Replikon aus der Kultur erhält.

27. Zelle, die ein Replikon nach einem der Ansprüche 1 bis 15 umfasst.

28. Zelle, die ein Virus nach einem der Ansprüche 19 bis 20 umfasst.

## Revendications

1. Réplicon inductible de virus de l'immunodéficience humaine dont la réplication est régulée par un agent exogène et qui comprend une séquence d'acides nucléiques, comprenant au moins un répresseur et/ou un activateur inductible, ainsi que toutes les séquences virales qui sont essentielles à la réplication sous le contrôle direct ou indirect dudit répresseur et/ou activateur inductible.

2. Réplicon inductible selon la revendication 1, dans lequel au moins une partie desdites séquences virales est de l'ARN.

3. Réplicon de virus selon la revendication 2, qui est dérivé d'un clone infectieux du virus de l'immunodéficience humaine.

4. Réplicon de virus selon l'une quelconque des revendications 1 à 3, dans lequel ledit répresseur et/ou activateur inductible comprend un opéron Tet ou un de ses équivalents fonctionnels.

5. Réplicon selon l'une quelconque des revendications 1 à 4, qui code pour un virus atténué.

6. Réplicon selon l'une quelconque des revendications 1 à 5, qui comprend en outre un gène suicide.

7. Réplicon selon l'une quelconque des revendications 1 à 6, qui peut être induit à se répliquer par la présence de doxycycline ou d'un de ses analogues fonctionnels.

8. Réplicon selon l'une quelconque des revendications 3 à 7, qui comprend en outre un élément TAR inactivé.

9. Réplicon selon l'une quelconque des revendications 3 à 8, qui comprend en outre un élément Tat inactivé.

10. Réplicon selon l'une quelconque des revendications 3 à 7, dans lequel au moins une partie fonctionnelle, de préférence un gène rtTA, dudit répresseur et/ou activateur inductible est introduite dans le gène nef.

11. Réplicon selon la revendication 10, dans lequel au moins une partie du gène nef est supprimée afin de créer un espace pour l'insertion.

12. Réplicon selon l'une quelconque des revendications 3 à 11, dans lequel au moins un élément NF-kB a été supprimé.

13. Réplicon selon l'une quelconque des revendications 3 à 12, qui comprend au moins un motif tetO dans au moins une LTR fonctionnelle.

14. Réplicon selon la revendication 13, qui comprend au moins 2, 4, 6 ou 8 d'éléments de ce type dans au moins une LTR fonctionnelle.

15. Réplicon selon l'une quelconque des revendications 3 à 14, dans lequel au moins une LTR est modifiée pour éviter la réversion d'un virus de type sauvage.

16. Procédé de production d'un virus de l'immunodéficience humaine dépendant d'un agent inducteur pour se répliquer, comprenant les étapes consistant à introduire le réplicon selon l'une quelconque des revendications 1 à 15 dans une cellule permissive, à cultiver ladite cellule en présence dudit agent inducteur et à récolter ledit virus dépendant à partir de ladite culture.

17. Procédé selon la revendication 16, dans lequel ledit virus est un virus atténué.

18. Procédé selon l'une quelconque des revendications 16 à 17, dans lequel ledit agent inducteur est la doxycycline ou un de ses analogues fonctionnels.

19. Virus dépendant d'un agent inducteur pour se répliquer, pouvant être obtenu par un procédé selon l'une quelconque des revendications 16 à 18.

20. Virus selon la revendication 19, qui est un virus atténué.

21. Vaccin comprenant un réplicon selon l'une quelconque des revendications 1 à 15 et/ou un virus selon l'une quelconque des revendications 19 à 20, une quantité d'agent inducteur et un adjuvant adéquat.

22. Vaccin selon la revendication 21, dans lequel ledit agent inducteur est fourni séparément.

23. Procédé de réplication contrôlée d'un virus ou d'un réplicon de virus, comprenant les étapes consistant à introduire dans une cellule permissive le réplicon selon l'une quelconque des revendications 1 à 15 ou le virus selon la revendication 19 ou 20, à cultiver ladite cellule en présence dudit agent inducteur et à manipuler la quantité d'agent inducteur présent.

24. Réplicon selon l'une quelconque des revendications 1 à 15 et/ou virus selon l'une quelconque des revendications 19 à 20, destiné à être utilisé comme agent prophylactique.

25. Utilisation d'un réplicon selon l'une quelconque des revendications 1 à 15 et/ou virus selon l'une quelconque des revendications 19 à 20 pour préparer un vaccin destiné à la prévention du SIDA.

26. Procédé de modification d'un réplicon inductible selon l'une quelconque des revendications 1 à 15, comprenant les étapes consistant à
- générer un réplicon de virus de l'immunodéficience humaine comprenant un acide nucléique qui code pour toutes les séquences virales qui sont essentielles à la réplication sous le contrôle direct ou indirect d'au moins un répresseur et/ou un activateur inductible.
- introduire ledit réplicon dans des cellules permissives pour la réplication dudit réplicon,
- cultiver lesdites cellules dans des conditions qui permettent la réplication dudit réplicon,
- obtenir un réplicon répliqué à partir de ladite culture.

27. Cellule comprenant un réplicon selon l'une quelconque des revendications 1 à 15.

28. Cellule comprenant un virus selon l'une quelconque des revendications 19 à 20.
